(19) Europäisches Patentamt
European Patent Office
Office européen des brevets

(11) EP 0 540 975 B1

(12) **EUROPÄISCHE PATENTSCHRIFT**

(45) Veröffentlichungstag und Bekanntmachung des Hinweises auf die Patenterteilung:
**10.01.1996 Patentblatt 1996/02**

(51) Int. Cl.$^6$: **C07C 229/16**, A61K 49/00

(21) Anmeldenummer: **92118289.5**

(22) Anmeldetag: **26.10.1992**

(54) **Diethylentriamin-Derivate und deren Verwendung zu diagnostischen und therapeutischen Zwecken**

Diethylenetriamine derivatives and their use for diagnostic and therapeutic purposes

Dérivés de diéthylènetriamine et leur utilisation pour des buts diagnostiques et thérapeutiques

(84) Benannte Vertragsstaaten:
**CH DE FR GB LI SE**

(30) Priorität: **06.11.1991 DE 4136489**

(43) Veröffentlichungstag der Anmeldung:
**12.05.1993 Patentblatt 1993/19**

(73) Patentinhaber: **BAYER AG**
**D-51368 Leverkusen (DE)**

(72) Erfinder: **Mikhail, Gamal, Dr.**
**W-5068 Odenthal (DE)**

(56) Entgegenhaltungen:
**EP-A- 0 305 320**
**EP-A- 0 367 223**
**WO-A-84/03698**
**WO-A-86/06384**
**WO-A-91/03200**
**US-A- 4 152 345**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 540 975 B1

## Beschreibung

Metalle komplexierende Verbindungen finden zahlreiche Anwendungen in der Analytik, Forschung und Medizin und Umwelttechnologie. Einige Beispiele sind die Zurückgewinnung, Abtrennung und selektive Trennung von Metallionen, die therapeutische Entfernung von toxischen oder radioaktiven Metallionen aus dem Körper und die Anwendung als Ionenträger. In vielen Fällen wird eine Bindung, vorzugsweise eine kovalente Bindung angestrebt zwischen der komplexierenden Verbindung und einem Makromolekül oder anderen Strukturen, die es ermöglicht, die Metallionen an eine bestimmte Wirkungsstelle zu bringen.

Besonders interessante Anwendungsgebiete sind in der Medizin und der Diagnostik zu finden. So ist eine Krebs-Therapie mit monoklonalen Antikörper-Metallkomplex-Kunjugaten beschrieben (s. Kozak et al., Trend in Biotechnology 4, 259-264 (1985) und Proc. Natl. Acad. Sci. USA 83, 474 (1986)).

Auch die Anwendung von Metallkomplexen in sog. Magnetic Resonance Imaging (= MRI) ist beschrieben (s. beispielsweise Magerstadt et al., Magnetic Resonance Imad. 3, 808-812 (1986)).

Lanthanid-Komplexe sind u. a. als "Reporter Groups" für diagnostische Zwecke beschrieben (s. z. B. Clinical Biochem. 21, 173-178 (1988) und EPA 298 939). Komplexbildner, die insbesondere Lanthanide binden, sind in der WO-A-84/03698 beschrieben.

Weitere Anwendungen für Metalle komplexierende Verbindungen und die entsprechenden Metallkomplexe können beispielsweise aus WO 90/09379, WO 90/08138, WO 90/08134, WO 89/01476, WO 89/11868, WO 89/11475, WO 89/05802 und WO 90/11282 entnommen werden. Die WO-A-91/03200 beschreibt Hydroxyaryl Metallchelate und deren Anwendung bei NMR Untersuchungen.

Für den Einsatz in der Medizin, Diagnostik und Analytik und Umwelttechnologie sollen die Komplexbildner vor allem eine hohe Komplexbildungskonstante (Ks) aufweisen. Außerdem sollen die Komplexbildner, Komplexe und Komplexsalze und ihre Konjugate folgende Anforderungen erfüllen:
Gute Wasserlöslichkeit, hohe Selektivität unter den Anwendungsbedingungen, keine Toxizität, Spezifizität für den Wirkungsort, gute Metallselektivität und verknüpfbare Struktur. Diese Anforderungen können durch Auswahl geeigneter Strukturteile beeinflußt werden.

Als Metalle komplexierende Verbindungen sind beispielsweise beschrieben die 4-Isothiocyanatobenzyl-Derivate der Diethylentriaminpentaessigsäure und der Ethylendiamintetraessigsäure (F. Keana et al., J. Org. Chem. 55, 2868-2871 (1990)). Die Verknüpfung an Makromoleküle und/oder Biomoleküle erfolgt dabei über die Reaktion mit der Isothiocyanat-Gruppe. Nachteilig bei diesen Verbindungen ist die schlechte Metallselektivität und die geringe Stabilität des Metallkomplexes.

Eine weitere komplexierende Verbindung, das N,N'-Bis-(2-Hydroxybenzyl)-1-(4-bromoacetamidobenzyl)-1,2-ethylendiamin-N,N'-diessigsaure ist durch die 4-Bromoacetamidobenzyl-Gruppe mit Antikörpern verknüpfbar (s. C.J. Mathias et al., Bioconjugate Chem. 1, 204-211 (1990)). Die Metall-Selektivität ist ausreichend, die Stabilität entsprechender Komplexe jedoch gering.

Der vorliegenden Erfindung liegt deshalb die Aufgabe zugrunde, verbesserte komplexierende Verbindungen, insbesondere Verbindungen mit verbesserter Komplexbildungsaffinität und gleichzeitig variablen Verknüpfungsmöglichkeiten und die entsprechenden Komplexe und/oder Komplexsalze zur Verfügung zu stellen.

Die vorliegende Erfindung betrifft Diethylentriamin-Derivate der Formel (I)

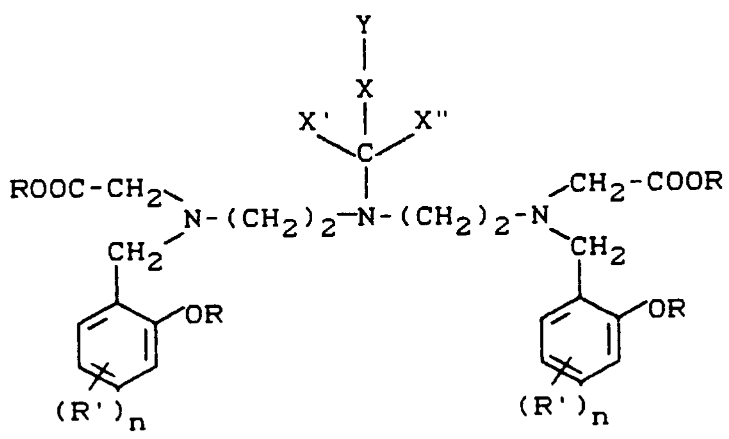

(I),

in der

X für gegebenenfalls Heteroatomgruppierungen enthaltendes $C_5$- bis $C_{14}$-Arylen oder $C_1$-bis $C_{20}$, Heteroatomgruppierungen enthaltendes (N, O, S (1 x, mehrfach)) Alkylen steht,

Y und gegebenenfalls X + Y für

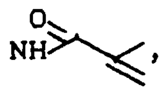,

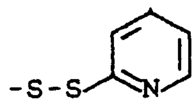,

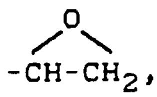,

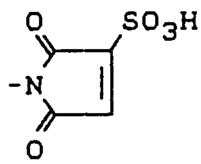,

-N-Oxysuccinimido, -N-Maleinsäureimido, $-NH_2$, -OH, $-COCH_2$-Halogen, Halogen,

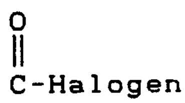,

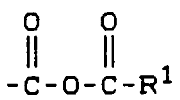, 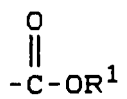, 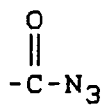, 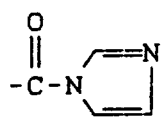

-NCO, -NCS, -CHO, -COOH, SH, oder $-CH=CH-CO_2R^1$

steht, wobei

$R^1$ und $R^2$ unabhängig voneinander jeweils für Wasserstoff, einen gesättigten oder ungesättigten, gegebenenfalls durch eine Phenylgruppe substituierten $C_1$- bis $C_{20}$-Alkylrest oder eine Phenylgruppe stehen,

X' und X" unabhängig voneinander jeweils für Wasserstoff, $C_1$-bis $C_{10}$-Alkyl oder $C_6$-bis $C_{10}$-Aryl stehen, wobei solche Alkyl- oder Arylreste gegebenenfalls mit einem weiteren Substituenten des Typs Y substituiert sein können,

R unabhängig voneinander jeweils für Wasserstoff, Ammonium oder ein Äquivalent eines Alkalimetalls oder 1/2 Äquivalent eines Erdalkalimetalls steht,

n unabhängig voneinander jeweils für Null, 1, 2, 3 oder 4 steht und

R' unabhängig voneinander jeweils für $C_1$-bis $C_4$-Alkyl, Chlor oder Brom steht.

Soweit X für Heteroatome enthaltendes Arylen steht, kommen als Heteroatome insbesondere Stickstoffatome in Frage. Einzelbeispiele für Heteroatome enthaltendes Arylen sind; Pyridylen, Pyrimidylen, Indolylen und Chinolylen.

Soweit Y $COCH_2$-Halogen oder Halogen bedeutet, kommen als Halogenatome insbesondere Chlor, Brom und Jod in Frage.

Soweit R für ein Äquivalent eines Alkalimetalls steht, kommen insbesondere Natrium oder Kalium in Frage, soweit R für 1/2 Äquivalent eines Erdalkalimetalls steht, insbesondere Magnesium und Calcium.

Vorzugsweise ist/sind

X ein kein Heteroatom enthaltendes $C_6$-bis $C_{10}$-Arylen oder $C_1$-bis $C_8$-Alkylen,

Y $-NH_2$, -OH, $-COCH_2$-Halogen, Halogen, -NCO, -NCS, -N-Oxysuccinimido, -N-Maleinsäureimido, -CHO, -COOH, SH oder

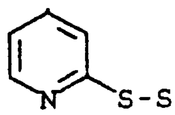

X' und X" gleich und bedeuten Wasserstoff, $C_1$-bis $C_6$-Alkyl oder Phenyl,

die Reste R gleich und bedeuten Wasserstoff, Natrium, Kalium, Magnesium oder Calcium,
die beiden n gleich und bedeuten Null, 1 oder 2 und alle R' gleich und bedeuten Methyl, Ethyl oder Chlor.

Besonders bevorzugt ist/sind

X Phenylen, Naphthylen, Methylen, Ethylen, n-Propylen, i-Propylen, n-Butylen oder i-Butylen,

Y Hydroxy, $NH_2$, SH, $CO_2H$, Halogen,

X' und X" gleich und bedeuten Wasserstoff, Methyl, Ethyl oder Phenyl,

die R gleich und bedeuten Natrium oder Kalium,
die beiden n gleich und bedeuten Null oder 1 und die beiden R' gleich und bedeuten Methyl oder Chlor.

Die erfindungsgemäßen Diethylentriamin-Derivate der Formel (I) können mit den verschiedensten Metallionen Komplexe bilden. Die vorliegende Erfindung bezieht sich auch auf solche Komplexe. Als zentrale Metallionen für solche Komplexe kommen beispielsweise Ionen von Elementen mit den Ordnungszahlen 21-29, 31, 32, 38, 39, 42-46, 48, 49 und 57-83 in Frage.

Je nachdem für welchen Zweck die Komplexe eingesetzt werden sollen sind gewisse zentrale Metallionen bevorzugt. Zur Anwendung in der MRI-Diagnostik sind zwei- und dreiwertige Ionen von Elementen mit den Ordnungszahlen 21-29, 42, 44 und 57-70 bevorzugt, insbesondere Chrom(III)-, Mangan(II)-, Eisen(II)-, Kobalt(II)-, Nickel(II)-, Kupfer(II)-, Praseodym(III)-, Neodym(III)-, Samarium(III)- und Ytterbium(III)-Ionen.

Wenn ein starkes magnetisches Moment gewünscht wird, dann sind Gadolinium(III)-, Terbium(III)-, Dysprozium(III)-, Holmium(III)-, Erbium(III)- und Eisen(III)-Ionen bevorzugt.

Zur Verwendung in der Nuklearmedizin können die zentralen Metallionen sich ganz oder teilweise von radioaktiven Elementen ableiten. Als Beispiele seien genannt: Radioisotope der Elemente Kupfer, Kobalt, Gallium, Germanium, Yttrium, Strontium, Technetium, Indium, Ytterbium, Gatolinium, Samarium und Iridium.

Zur Anwendung in der Röntgen-Diagnostik sind insbesondere solche zentralen Metallionen geeignet, die sich von einem Element höherer Ordnungszahl ableiten, beispielsweise von einem Lantaniden-Element.

Die erfindungsgemäßen Diethylentriamin-Derivate können auf an sich bekannte Weise ausgehend von Diethylentriamin hergestellt werden (siehe z.B. Bioconjugate Chem. 1, 201-204 (1990) und Austr. J. Chem. 32, 519 (1979)).

Die verschiedenen zentralen Metallionen können beispielsweise eingeführt werden, indem man die Stammverbindung der Formel (I) mit allen R = Wasserstoff einem beliebigen Salz oder Oxid des Metalls umsetzt.

Die Diethylentriamin-Derivate der Formel (I) können für die verschiedensten diagnostischen und therapeutischen Zwecke verwendet werden, beispielsweise zur Fluoreszenz- und Radiomarkierung von Biomolekülen (z.B. Proteinen, Nukleinsäuren, Antikörpern) und Wirkstoffen, in der Nuklearmedizin, beim sogenannten Magnetic resonance imaging und bei der Therapie mit Metallsalzen.

Bei den Markierungsverfahren kann über die Y-Gruppe eine Verknüpfung (chemische Bindung) zum jeweiligen Biomolekül oder Wirkstoff erfolgen.

Die erfindungsgemäßen Diethylentriamin-Derivate haben den Vorteil, daß sie aufgrund der Molekülgeometrie stabile Metallkomplexe darstellen, wobei hinsichtlich der Auswahl der Verknüfungsgruppe Y große Flexibilität besteht.

Beispiel 1

1,7-Diphthaloyldiethylentriamin: (1)

192 g (1,3 mol) Phthalsäureanhydrid werden in 2 l siedenem Chloroform vollständig gelöst. 62 g (0,6 mol) Diethylentriamin werden bei 50°C in 80 Minuten und unter starkem Rühren zugegeben. Das Reaktionsgemisch wird gelb und ein heller Niederschlag bildet sich. Das Chloroform wird abdestilliert und der Rückstand wird bei 135°C für 2 Stunden erhitzt. Das Rohprodukt wird pulverisiert, in minimale Mengen Chloroform gelöst und heiß filtriert. Das Produkt wird unter Zugabe der dreifachen Menge Ethanol ausgefällt, abfiltriert, getrocknet und umkristallisiert aus Chloroform-Ethanol. Nach dem Trocknen bei 120°C erhält man 135 g (61 % der Theorie) eines weißen Feststoffs vom Schmelzpunkt 175-176°C (Lit. = 180°C).

Beispiel 2a)

4-(p-Nitrobenzyl)-1,7-diphthaloyldiethylentriamin (2a)

9,6 g KOH (168 mmol) werden in 100 ml heißem Ethanol vollständig gelöst. 60 g (168 mmol) der in Beispiel 1 erhaltenen Verbindung 1 werden bei Raumtemperatur eingetragen und erhitzt unter Rückfluß für 2,5 Stunden. Eine trübe Mischung entsteht. 37,5 g (168 mmol) p-Nitrobenzylbromid werden unter starkem Rühren zugegeben und weiter unter Rückfluß für 16 Stunden erhitzt. Die trübe Lösung wird heiß filtriert. Nach abkühlen des Filtrats wird der entstandene Rückstand abgetrennt und in Vakuum getrocknet. Man erhält 66 g (80 % der Theorie) eines weißen Feststoffs vom Schmelzpunkt 130-131°C (Lit. = 129-130°C).

Beispiele 2b) bis 2c)

Die Verbindungen in den folgenden Beispielen 2b, 2c werden durch N-Alkylierung von 1,7-Diphthaloyldiethylentriamin (1) mit dem entsprechenden Alkylhalogenid analog Beispiel 2a) hergestellt.

Beispiel 2b)

4-(p-Brombenzyl)-1,7-diphthaloyldiethylentriamin (2b)

p-Brombenzylbromid wird eingesetzt. Man erhält in 65 %. der Theorie ein weißer Feststoff vom Schmelzpunkt 149-150°C.

| | |
|---|---|
| $^1$H-NMR: (in DMSO-$d_6$): | 7,85 m 4H, 7,7 m 4H, 6,95 s 4H, 3,6 t (j = 5Hz) 4H, 3,53 s 2H, 2,67 t (j = 5Hz) 4H. |
| MS : | $M^+$ = 531 |
| IR : | 3457 bw, 2945 w, 2834 w, 1770 w, 1710 ws, 1376 s, 1058 m. |

Beispiel 2c)

4-(3-Hydroxypropyl)-1,7-diphthaloyldiethylentriamin (2c)

3-Iodopropanol wird eingesetzt. Man erhält einen weißen Feststoff vom Schmelzpunkt 111-112°C.

| | |
|---|---|
| $^1$H-NMR; (in $CDCl_3$); | 7,7 m 8H, 3,8 t (j = 6Hz) 4H, 3,5 t (j = 5Hz) 2H, 2,83 t (j = 6Hz) 4H, 2,62 t (j = 5Hz) 2H, 1,1 n 2H. |
| MS (CI): | $M^+$ = 421. |

Beispiel 3a)

4-(p-Nitrobenzyl)-diethylentriamin (3a)

46 g (92 mmol) der in Beispiel 2a) beschriebenen Bisphthalimid werden in 0,8 l 6N HCl suspendiert und zum Sieden erhitzt. Die klare Lösung wird nach 12 Stunden Rückfluß abgekühlt, dabei scheiden sich weiße Kristalle ab. Anschließend wird filtriert und in Vakuum eingeengt. Der weiße Rückstand wird in 50 ml 2N NaOH aufgenommen und mit Chloroform 3 mal 200 ml extrahiert. Nach Trocknen der organischen Phase über Natriumsulfat wird in Vakuum eingeengt.

Man erhält 14,6 g (67 % der Theorie) eines gelben Öls.

$^1$H-NMR: (in $CDCl_3$): 8,2 d (j = 10Hz) 2H, 7,5 d (j = 10Hz) 2H, 3,72 s 2H, 2,8 (t, j = 6,5Hz) 4H, 2,55 t (j = 6,5Hz) 4H, 1,8 bs 4H.

Beispiel 3b)

4-(p-Brombenzyl)-diethylentriamin (3b)

10 g (18,8 mmol) der in Beispiel 2b) beschriebenen Bisphthalimid werden analog der für Beispiel 3a) gegebenen Vorschrift zu 2,6 g (51 % der Theorie) der Titelverbindung umgesetzt.

$^1$H-NMR: 7,95 d (j = 9,5Hz) 2H, 7,2 d (j = 9,5) 2H, 3,6 s 2H, 2,78 t (j = 6Hz) 4H, 2,64 t (j = 6Hz) 4H, 1,65 bs 4H

MS (CI): $M^+$ = 272

Beispiel 4

1,7-Bis-(salicyliden)-4-(p-nitrobenzyl)-diethylentriamin (4)

14 g (48 mmol) der in Beispiel 3a) beschriebenen Diamin werden in 500 ml abs. Ethanol gelöst und mit 14,4 g (118 mmol) Salicylaldehyd versetzt. Nach 20 Stunden Rühren bei Raumtemperatur wird die klare gelbe Lösung eingeengt. Der Rückstand wird in 250 ml warmes Ethanol gelöst und über Nacht bei -4°C gestellt.

Man erhält 12,5 g (58 % der Theorie) eines gelben Feststoffs vom Schmelzpunkt 91-93°C (Lit. 86-88°C).

$^1$H-NMR; (in $CDCl_3$): 13,3 bs 2H, 8,25 s 2H, 7,95 dt (j = 9 und 1Hz) 2H, 7,25-7,45 m 4H, 6,8-7,1 m 6H, 3,8 s 2H, 3,7 t 4H, 2,8 t 4H.

MS (CI): $M^+$ = 446

Beispiel 5

1,7-Bis-(2-hydroxyphenyl)-4-(p-nitrobenzyl)-diethylentriamin (5)

6 g (13,5 mmol) der in Beispiel 4) beschriebenen Schiffsche-Base werden in 200 ml abs. Ethanol gelöst und mit 1 g (27 mmol) Natriumhydrid versetzt. Die Reaktion ist leicht exotherm. Nach 2 Stunden Rühren bei Raumtemperatur wird die trübe Lösung filtriert und mit Ethanol gewaschen. Man erhält 4,5 g (75 % der Theorie) eines weißen Feststoffs. Nach umkristallisieren aus warmen Ethanol entstehen 2,5 g eines Feststoffs vom Schmelzpunkt 108-109°C (Lit. 108-110°C).

Beispiel 6

1,7-Bis-(2-hydroxyphenyl)-4-(p-nitrobenzyl)-diethylentriamin-1,7-diessigsäurediethylester (6)

1 g (2,24 mmol) der in Beispiel 5) beschriebenen Diamin wird in 60 ml trockenem Tetrahydrofuran gelöst und mit 1,2 g (7,32 mmol) Bromessigsäureethylester und 0,9 g (9 mmol) Triethylamin versetzt. Die klare Lösung wird bei 40°C für 48 Stunden gerührt. Nach dem Abkühlen auf 0°C und anschließenden Filtrieren wird die Lösung im Vakuum eingeengt und auf Kieselgel chromatographiert. (Laufmittel: Chloroform : Methanol : Acetonitril = 98:1:1).

Man erhalt 856 mg (63 % der Theorie) eines gelben Öls.

$^1$H-NMR: (in $CDCl_3$): 9,7-9,8 bs 2H, 8,1 d (j = 8,5Hz) 2H, 7,38 d (j = 8,5Hz) 2H, 7,2 dt (j = 8 und 1Hz) 2H, 6,8-7 m 6H, 4,17 q (j = 7Hz) 4H, 3,72 s 4H, 3,52 s 2H, 3,25 s 4H, 2,5-2,8 m 8H, 1,22 t (j = 7Hz) 6H.

MS (CI): $M^+$ = 530

Beispiel 7

1,7-Bis(2-hydroxybenzyl)-4-(p-nitrobenzyl)-diethylentriamin-1,7-diessigsäuredi-t-butylester (7)

6 g (13,2 mmol) der in Beispiel 5) beschriebenen Daimin werden in 120 ml trockenen Tetrahydrofuran gelöst und mit 5.16 g (26,4 mmol) Bromessigsäure-t-butylester und 3 g (29,4 mmol) Triethylamin versetzt. Die klare Lösung wird bei 40°C für 20 Stunden gerührt. Bromessigsäure-t-butylester sowie Triethylamin werden weiter zugegeben bis das Diamin vollständig abreagiert. Nach dem Abkühlen und anschließendem Filtrieren wird die Lösung im Vakuum eingeengt und auf Kieselgel chromatographiert.

Man erhält 6,6 g (73 % der Theorie) eines schwach gelben Feststoffes vom Schmelzpunkt 105-107°C.

| | |
|---|---|
| $^1$H-NMR (in $CDCl_3$): | 9,9-9,7 bs 2H; 8,1 d (j = 8,5 Hz) 2H; 7,35 d (8,5 Hz); 7,2 dt (j = 8 und 1 Hz) 2H; 6,7-7 m 6H; 3,73 s 4H; 3,57 s 2H; 3,18 s 4H; 2,5-2,7 m 8H; 1,43 s 18 H |
| MS (CI): | $M^+$ = 678 |

Beispiel 8

1,7-Bis-(2-hydroxybenzyl)-4-(p-nitrobenzyl)-diethylentriamin-1,7-diessigsäure (8)

i- aus dem Triamin 5 durch N-Alkylierung:
1 g (2,24 mmol) der in Beispiel 5) beschriebenen Triamin wird in 60 ml trockenem THF gelöst und mit 1 g (7,3 mmol) Bromessigsäure und 0,9 g (9 mmol) Triethylamin versetzt. Die klare Lösung wird nach 6 Stunden bei 40°C trübe. Nach 30 Stunden bei 40°C wird die Lösung in Vakuum eingeengt und über Ionenaustauscher gereinigt. Man erhält 1,1 g (81 % der Theorie) eines weißen Feststoffs.
Die Lösung wird eingeengt und der Rückstand über Ionenaustauscher gereinigt. Man erhält 350 mg (77 % der Theorie) eines weißen Feststoffs.

ii - aus dem Di-t-butylester 7 durch Esterspaltung:
0,5 g (0,8 mmol) der in Beispiel 7) beschriebenen Diester wurden in 25 ml Benzol gelöst und mit 4,8 ml (42 mmol) Trifluoressigsäure versetzt. Nach 2 Stunden Rühren bei 60°C ist die Reaktion beendet (lt. DC, Laufmittel Chloroform : Ethanol = 9 : 1).

Beispiel 9

Stabilitätskonstante (Ks) von Europiumkomplexen mit dem Komplexbildner 8

1. Vergleichssubstanzen:
Als Vergleich wurde eine Reihe von Komplexbildnern hergestellt. 2,6-disubstituierte Pyridinderivate bilden besonders stabile Komplexe mit Europium (EPA 298 939). 4-Chlor-2,6-bis[N,N-bis(carboxymethyl)-aminomethyl]pyridin (9) wurde nach Vögtle und Ohm in Chem. Ber. 117, 948 (1984) hergestellt.

2. Europiumkomplexe:
Die Europiumkomplexe von 8 bzw. 9 wurden aus den entsprechenden Na-Salzen hergestellt. Zur Herstellung der Na-Salze wurden die Komplexbildner 8 bzw. 9 mit 4 Molequivalenten Natriumhydroxid in wäßriger Lösung versetzt und anschließend im Vakuum eingeengt.

3. Stabilitätskonstante (Ks):
Die Gleichgewichtskonstante (K) bei der Bildung eines Europiumkomplexes ($EuL^-$) aus dem Europiumion ($Eu^{3+}$) und den Liganden 8 bzw. 9 ($L^{4-}$) heißt Stabilitätskonstante Ks und wird aus dem Massenwirkungsgesetz wie folgt ermittelt:

$$K_s = \frac{c(EuL^-)}{c(L^{4-}) \cdot c(Eu^{3+})}$$

Europium(III)chlorid-hexahydrat und der betreffende Ligand wurden in einem definierten Massenverhältnis in einem definierten Volumen Wasser gelöst. Die Konzentration an nicht komplexgebundenem Europium wurde bei pH = 4,65 durch Titration mit EDTA-Messlösung gegen Xylenolorange als Indikator bestimmt. Der EDTA-Komplex ist schwächer als die hier zu untersuchenden Komplexe, so daß selektiv nur das freie $Eu^{3+}$ erfaßt wird.

$K_s$ für 9 = 1,47 x $10^2$ $Mol^{-1}$.L
$K_s$ für 8 = ∞ (unbestimmbar)*

## Patentansprüche

1. Diethylentriamin-Derivate der Formel (I)

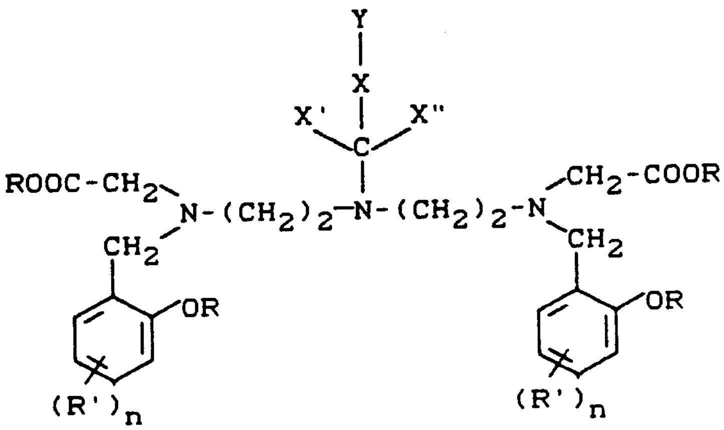

(I),

in der

X für gegebenenfalls Heteroatomgruppierungen enthaltendes $C_6$- bis $C_{14}$-Arylen oder $C_1$-bis $C_{20}$, Heteroatomgruppierungen enthaltendes (N, O, S (1 x, mehrfach)) Alkylen steht,

Y für

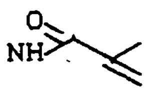, 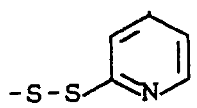,

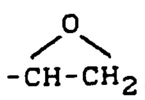, 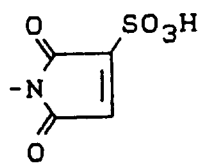,

-N-Oxysuccinimido, -N-Maleinsäureimido, $-NH_2$, -OH, $-COCH_2$-Halogen, Halogen, -NCO, -NCS, -

*) Da kein freies Europiumion nachgewiesen werden konnte. Die Nachweisgrenze der Methode liegt bei ungefähr 3 x $10^{-5}$M.

CHO, -COOH, SH,

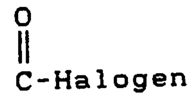,

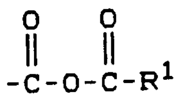, 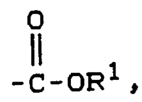, 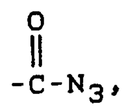, 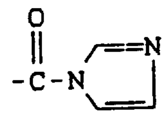

oder
$-CH=CH-CO_2R^1$
steht, wobei

$R^1$ und $R^2$ unabhängig voneinander jeweils für Wasserstoff, einen gesättigten oder ungesättigten, gegebenenfalls durch eine Phenylgruppe substituierten $C_1$- bis $C_{20}$-Alkylrest oder eine Phenylgruppe stehen,

X' und X" unabhängig voneinander jeweils für Wasserstoff, $C_1$-bis $C_{10}$-Alkyl oder $C_6$-bis $C_{10}$-Aryl stehen, wobei solche Alkyl- oder Arylreste gegebenenfalls mit einem weiteren Substituenten des Typs Y substituiert sein können,

R unabhängig voneinander jeweils für Wasserstoff, Ammonium oder ein Äquivalent eines Alkalimetalls oder 1/2 Äquivalent eines Erdalkalimetalls steht,

n unabhängig voneinander jeweils für Null, 1, 2, 3 oder 4 steht und

R' unabhängig voneinander jeweils für $C_1$- bis $C_4$-Alkyl, Chlor oder Brom steht,

**2.** Verbindungen gemäß Anspruch 1, wobei X für Pyridylen, Pyrimidylen, Indolylen und Chinolylen steht.

**3.** Verbindungen gemäß Anspruch 1, wobei X ein kein Heteroatom enthaltendes $C_6$-bis $C_{10}$-Arylen oder $C_1$- bis $C_8$-Alkylen bedeutet.

**4.** Verbindungen gemäß einem der Ansprüche 1 bis 3, wobei Y $-NH_2$, -OH, $-COCH_2$-Halogen, Halogen, -NCO, -NCS, -N-Oxysuccinimido, -N-Maleinsäureimido, -CHO,
-COOH, SH oder

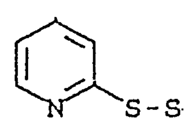

bedeutet.

**5.** Verbindungen gemäß einem der Ansprüche 1 bis 4, wobei X' und X" gleich sind und bedeuten Wasserstoff, $C_1$-bis $C_6$-Alkyl oder Phenyl.

**6.** Verbindungen gemäß einem der Ansprüche 1 bis 5 wobei die Reste R gleich sind und bedeuten Wasserstoff, Natrium, Kalium, Magnesium oder Calcium.

**7.** Verwendung der Verbindungen gemäß einem der Ansprüche 1 bis 6 als Komplexbildner in der Diagnostik, in der Analytik, in der Umwelttechnologie und bei chemischen Reinigungsverfahren.

**8.** Verwendung der Verbindungen gemäß einem der Ansprüche 1 bis 6 zur Herstellung von Arzneimitteln.

## Claims

1. Diethylenetriamine derivatives of the formula (I)

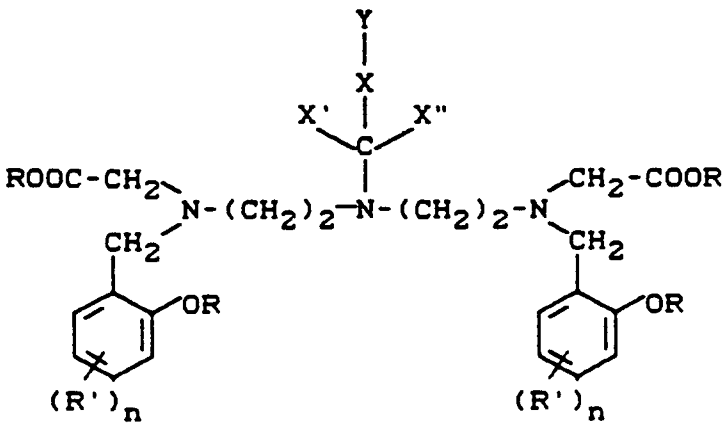

(I)

in which

X represents $C_6$- to $C_{14}$-arylene which optionally contains hetero atom groupings, or $C_1$- to $C_{20}$-alkylene which contains hetero atom groupings (N, O, S (1 ×, two or more times)),

Y represents

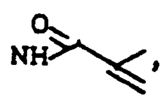, 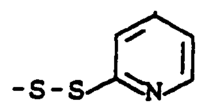,

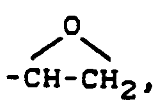, 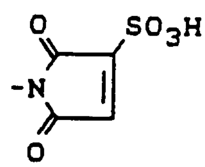,

-N-oxysuccinimido -N-maleimido, $-NH_2$, -OH, $-COCH_2$-halogen, halogen, -NCO, -NCS, -CHO,

-COOH, SH, $\overset{O}{\overset{\|}{C}}$-halogen,

$-\overset{O}{\overset{\|}{C}}-O-\overset{O}{\overset{\|}{C}}-R^1$, $-\overset{O}{\overset{\|}{C}}-OR^1$, $-\overset{O}{\overset{\|}{C}}-N_3$, 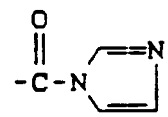

or $-CH=CH-CO_2R^1$, wherein

$R^1$ and $R^2$ independently of one another each represent hydrogen, a saturated or unsaturated $C_1$- to $C_{20}$-alkyl radical which is optionally substituted by a phenyl group, or a phenyl group,

X' and X" independently of one another in each case represent hydrogen, $C_1$- to $C_{10}$-alkyl or $C_6$- to $C_{10}$-aryl, it being possible for such alkyl or aryl radicals to be optionally substituted with a further substituent of the type Y,

R independently in each case represents hydrogen, ammonium or one equivalent of an alkali metal or 1/2 equivalent of an alkaline earth metal,

n independently in each case represents zero, 1, 2, 3 or 4 and

R' independently in each case represents $C_1$- to $C_4$-alkyl, chlorine or bromine.

2. Compounds according to Claim 1, wherein X represents pyridylene, pyrimidylene, indolylene or quinolylene.

3. Compounds according to Claim 1, wherein X denotes a $C_6$- to $C_{10}$-arylene or $C_1$- to $C_8$-alkylene which contains no hetero atom.

4. Compounds according to one of Claims 1 to 3, wherein Y denotes $-NH_2$, -OH, $-COCH_2$-halogen, halogen, -NCO, -NCS, -N-oxysuccinimido, -N-maleimido, -CHO, -COOH, SH or

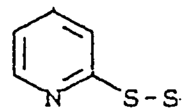

5. Compounds according to one of Claims 1 to 4, wherein X' and X" are identical and denote hydrogen, $C_1$- to $C_6$-alkyl or phenyl.

6. Compounds according to one of Claims 1 to 5, wherein the radicals R are identical and denote hydrogen, sodium, potassium, magnesium or calcium.

7. Use of the compounds according to one of Claims 1 to 6 as complexing agents in diagnostics, in analysis, in environmental technology and in chemical cleaning processes.

8. Use of the compounds according to one of Claims 1 to 6 for the preparation of medicaments.

## Revendications

1. Dérivés de diéthylènetriamine de formule (I)

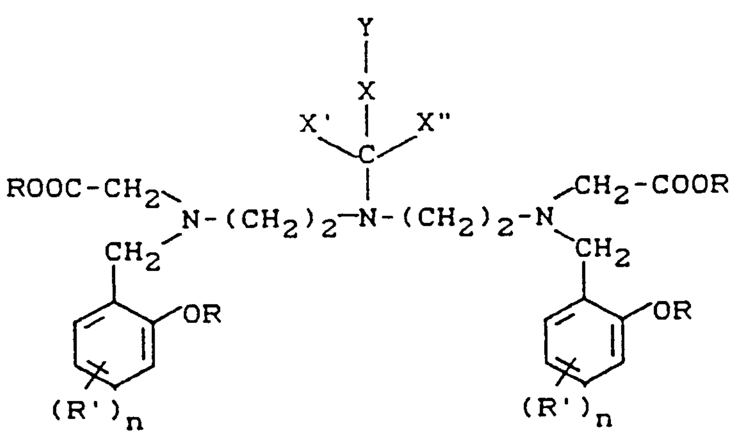

(I),

dans laquelle

X représente un groupe arylène en $C_6$ à $C_{14}$ contenant éventuellement des groupements hétéroatomiques ou un groupe alkylène en $C_1$ à $C_{20}$ contenant des groupements hétéroatomiques (N, O, S (1 x, multiple)),

Y représente

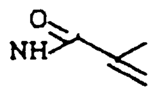, 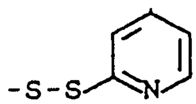,

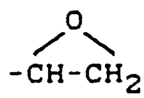, 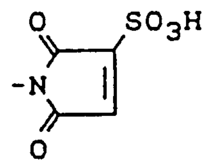,

un groupe -N-oxysuccinimido, N-maléimido, $-NH_2$, -OH, $-COCH_2$-halogène, un halogène, un groupe

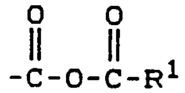, 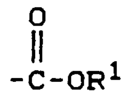, 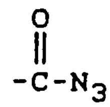, 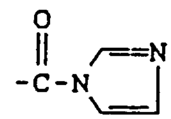

ou

$-CH=CH-CO_2R^1$

où

- $R^1$ et $R^2$ représentent chacun, indépendamment l'un de l'autre, de l'hydrogène, un reste alkyle en $C_1$ à $C_{20}$, saturé ou non saturé, portant éventuellement un substituant phényle, ou un groupe phényle,
- X' et X" représentent chacun, indépendamment l'un de l'autre, de l'hydrogène, un reste alkyle en $C_1$ à $C_{10}$ ou un reste aryle en $C_6$ à $C_{10}$, ces restes alkyle ou aryle pouvant être substitués le cas échéant avec un autre substituant du type Y,
- les restes R représentent chacun, indépendamment les uns des autres, de l'hydrogène, l'ion ammonium ou un équivalent d'un métal alcalin ou un demiéquivalent d'un métal alcalino-terreux,
- les indices n ont, indépendamment l'un de l'autre, chacun la valeur 0, 1, 2, 3 ou 4 et
- les restes R' représentent chacun, indépendamment l'un de l'autre, un reste alkyle en $C_1$ à $C_4$, du chlore ou du brome.

2. Composés suivant la revendication 1, dans lequel X est un groupe pyridylène, pyrimidylène, indolylène ou quinolylène.

3. Composés suivant la revendication 1, dans lequel X est un groupe arylène en $C_6$ à $C_{10}$ ou un groupe alkylène en $C_1$ à $C_8$ ne contenant pas d'hétéroatome.

4. Composés suivant l'une des revendications 1 à 3, dans lesquels Y est un groupe $-NH_2$, -OH, $-COCH_2$-halogène, un halogène, un groupe -NCO, -NCS, -N-oxysuccinimido, -N-maléimido, -CHO
-COOH, SH ou

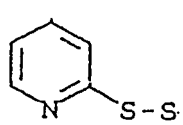

5. Composés suivant l'une des revendications 1 à 4, dans lesquels X' et X" sont égaux et désignent de l'hydrogène, un groupe alkyle en $C_1$ à $C_6$ ou un groupe phényle.

6. Composés suivant l'une des revendications 1 à 5, dans lesquels les restes R sont égaux et désignent de l'hydrogène, du sodium, du potassium, du magnésium ou du calcium.

7. Utilisation des composés suivant l'une des revendications 1 à 6, comme complexants dans le diagnostic, dans l'analyse, dans la technologie de l'environnement et dans des procédés chimiques d'épuration.

8. Utilisation des composés suivant l'une des revendications 1 à 6 pour la préparation de médicaments.